# EUROPEAN PATENT APPLICATION

(11) **EP 4 613 743 A1**
(43) Date of publication of application: **10.09.2025**
(21) Application number: 23912011.6
(22) Date of filing: 22.12.2023
(51) Int. Cl.: C07D 341/00, A61K 31/385, A61K 31/496, A61K 47/69, A61P 29/00, C08B 37/16

(54) **TRISULFIDE COMPOUND AND CLATHRATE THEREOF**

(30) Priority: 27.12.2022 JP 2022210346
(71) Applicant: Kyowa Pharma Chemical Co., Ltd., Takaoka-shi, Toyama 933-8511 (JP)
(72) Inventor: TOMONAGA, Shoichiro, Takaoka-shi, Toyama 933-8511 (JP); ISHIMARU, Hiroaki, Takaoka-shi, Toyama 933-8511 (JP); OHSHIMA, Etsuo, Tokyo 164-0001 (JP)
(74) Representative: Vossius & Partner Patentanwälte Rechtsanwälte mbB
(86) International application number: PCT/JP2023/046272
(87) International publication number: WO 2024/143231

(57) **Abstract**

Disclosed is a compound represented by formula (1) or a salt thereof: [Formula 1] [In the formula, R¹ is a group represented by formula (2), a group represented by formula (3), a group represented by formula (4), or a group represented by formula (5)] [Formula 2] [In the formula, R² and R³ each independently are a C1 to 4 alkylene group and * represents a bond] [Formula 3] [In the formula, R⁴ and R⁵ each independently represent a hydrogen atom or an optionally substituted C1 to 4 alkyl group, R⁴ and R⁵ may form a ring together with the atoms to which they are bonded, and * represents a bond] [Formula 4] [In the formula, * represents a bond] [Formula 5] [In the formula, * represents a bond].

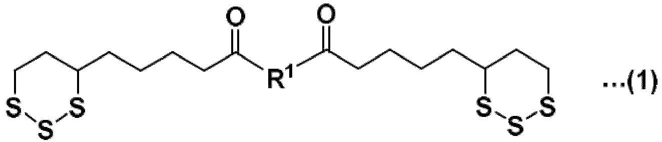

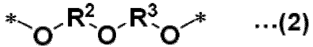

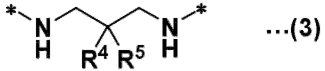

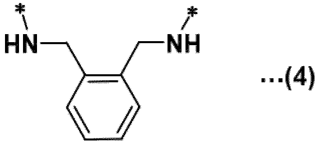

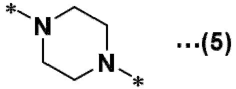

## Description

### Technical Field

The present invention relates to a trisulfide compound and a clathrate thereof.

### Background Art

A compound having a covalent bond structure consisting of three consecutive sulfur atoms is called a trisulfide compound. Trisulfide compounds are expected to have various physiologically active functions because they possess redox capability depending on the possible valence states of the constituent sulfur atoms.

Patent Literature 1 discloses lipoic acid trisulfide, a trisulfidated form of α-lipoic acid. Lipoic acid has been used for the treatment of diabetes or chronic hepatitis. Lipoic acid trisulfide can be produced, for example, by the methods described in Patent Literatures 2 and 3.

Non-Patent Literature 1 demonstrates that administration of polysulfides alleviates neurodegeneration in a Parkinson's disease model mouse. Non-Patent Literature 1 discloses glutathione trisulfide and lipoic acid trisulfide as examples of polysulfides.

Although trisulfide compounds have various physiologically active functions and are expected to be applied to novel medical uses, there are recognized issues for practical application, such as water solubility and stability required for active pharmaceutical ingredients. For example, Patent Literature 3 discloses a method for improving water solubility. Non-Patent Literature 2 describes a trisulfide compound having high water solubility. In addition, Non-Patent Literature 3 reports on the stability of polysulfide compounds including trisulfides and improvements thereof.

### Citation List

### Patent Literature

[Patent Literature 1] CN 107652264
[Patent Literature 2] WO 2021/200487
[Patent Literature 3] WO 2022/045212

### Non Patent Literature

[Non-Patent Literature 1] F. Nagashima et al., "Sulfide: quinone oxidoreductase ameliorates neurodegeneration in a murine model of Parkinson's disease", Redox Biology, 59, 2023, 102562
[Non-Patent Literature 2] E. Kanemaru et al., "Intranasal administration of polysulfide prevents neurodegeneration in spinal cord and rescues mice from delayed paraplegia after spinal cord ischemia", Redox Biology, 60, 2023, 102620
[Non-Patent Literature 3] T. Sawa et al., "Chemical Biology of Reactive Sulfur Species: Hydrolysis-Driven Equilibrium of Polysulfides as a Determinant of Physiological Functions", Antioxidants & Redox Signaling, 36, 2022, 327

### Summary of Invention

### Technical Problem

An object of the present invention is to provide a novel trisulfide compound and a cyclodextrin clathrate thereof (hereinafter also referred to as a "CD clathrate" or simply "clathrate").

### Solution to Problem

The present inventors have conducted intensive studies from the viewpoint of the structure and stability of trisulfide compounds and have found a novel compound having high stability. The present invention relates, for example, to the following [1] to [4].
[1] A compound represented by the Formula (1) or a salt thereof: 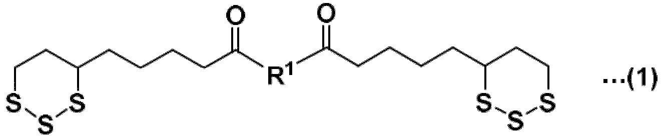
   wherein R¹ is a group represented by the Formula (2): 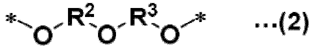
   wherein R² and R³ are each independently a C₁₋₄ alkylene group, and * is a bond;
   a group represented by the Formula (3): 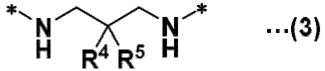
   wherein R⁴ and R⁵ are each independently a hydrogen atom, a C₁₋₄ alkyl group optionally having a substituent, R⁴ and R⁵ may form a ring together with the atom bonded thereto, and * is a bond;
   a group represented by the Formula (4): 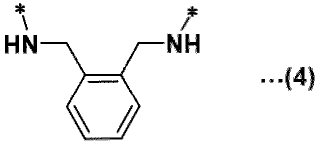
   wherein * is a bond; or
   a group represented by the Formula (5): 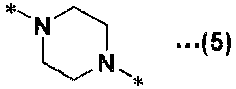
   wherein * is a bond.
[2] A cyclodextrin clathrate wherein the compound or salt thereof according to [1] is included in a cyclodextrin.
[3] The clathrate according to [2], wherein the salt of the compound represented by Formula (1) comprises at least one selected from the group consisting of a salt with an alkali metal, a salt with an alkaline earth metal, an ammonium salt, a salt with an inorganic acid, and a salt with an organic acid.
[4] The clathrate according to [2] or [3], wherein the cyclodextrin is at least one selected from the group consisting of α-cyclodextrin, β-cyclodextrin, γ-cyclodextrin, and a derivative thereof.

### Advantageous Effects of Invention

According to the present invention, it is possible to provide a novel trisulfide compound and a CD clathrate thereof. Lipoic acid trisulfide is considered to exhibit reactive oxygen species-eliminating action, hydrogen sulfide-eliminating action, and the like, derived from its trisulfide structure. The trisulfide compound of the present invention is expected to further enhance these actions because it contains two trisulfide structures in the molecule. In addition, the cyclic trisulfide structure is also expected to exhibit scavenging action against radicals which is biologically detrimental. The trisulfide compound according to the present invention is expected to more effectively terminate radical chain reactions by arranging two cyclic trisulfide structures in the molecule. Since radical chain reactions can also be a factor causing destabilization of the trisulfide structure, if the arrangement of two cyclic trisulfide structures in the molecule can effectively terminate radical chain reactions, it is also expected to delay the rate of decrease of the active ingredient due to decomposition in forms used for pharmaceutical applications (solid, powder, solution, etc.). Furthermore, the trisulfide compound of the present invention has a hydrogen bond and/or hydrophilic structure in the linker portion connecting the two lipoic acid trisulfide structures, thereby improving water solubility and providing a compound with an excellent balance of water solubility and lipophilicity. The trisulfide compound of the present invention is considered to have stability equal to or greater than that of lipoic acid trisulfide. The trisulfide compound of the present invention has better thermal stability than lipoic acid trisulfide.

The CD clathrate of the present invention is also considered to have improved reactive oxygen species-eliminating action, hydrogen sulfide-eliminating action, radical scavenging action, and the like, similar to the trisulfide compound of the present invention. Furthermore, by forming a CD clathrate of the trisulfide compound of the present invention, water solubility and stability are further improved.

### Brief Description of Drawings

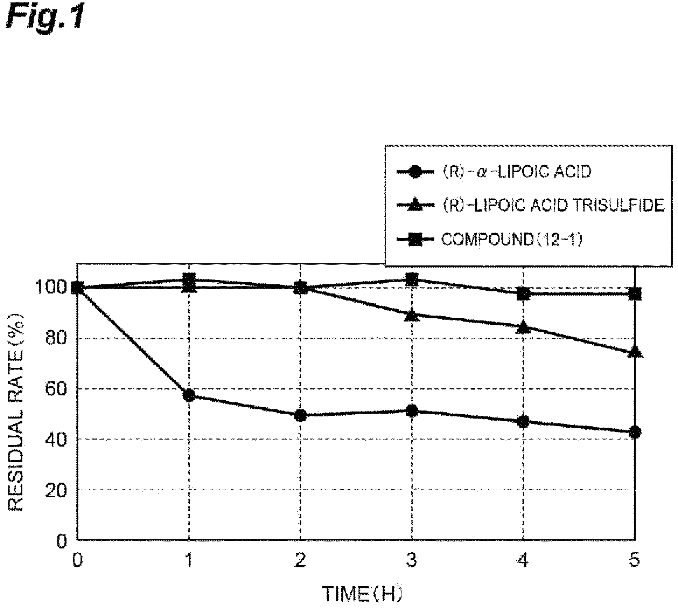

FIG. 1 is a graph showing the results of the thermal stability test in Test Example 1.

### Description of Embodiments

A trisulfide compound according to one embodiment of the present invention is compound (1). Compound (1) can be described as a compound in which two molecules of lipoic acid trisulfide are linked via a linker.

R² and R³ are each independently a C₁₋₄ alkylene group. Examples of C₁₋₄ alkylene groups include methylene, ethylene, n-propylene, isopropylene, cyclopropylene, n-butylene, isobutylene, sec-butylene, tert-butylene, and cyclobutylene.

R⁴ and R⁵ are each independently a hydrogen atom or a C₁₋₄ alkyl group optionally having a substituent, and R⁴ and R⁵ may form a ring together with the atom to which they are bonded. Examples of substituents include a hydroxy group; C₁₋₄ alkoxy groups such as methoxy, ethoxy, propoxy, and butoxy. Examples of C₁₋₄ alkyl groups include methyl, ethyl, n-propyl, isopropyl, cyclopropyl, n-butyl, isobutyl, sec-butyl, tert-butyl, and cyclobutyl. When R⁴ and R⁵ form a ring together with the atom to which they are bonded, the formed ring may be, for example, an oxetane ring.

Compound (1) can be produced by:
(A) a step of condensing a compound represented by Formula (1a) (lipoic acid trisulfide) with a molecule for forming a linker (a compound represented by Formula (2a), a compound represented by Formula (3a), o-xylylenediamine, m-xylylenediamine, p-xylylenediamine, piperazine, etc.) (Step 1), or
(B) a step of condensing a compound represented by Formula (1b) (lipoic acid) with a molecule for forming a linker as described above (Step 1'), a step of oxidizing the obtained disulfide compound with an oxidizing agent to obtain a sulfoxide compound (Step 2), and a step of reacting the obtained sulfoxide compound with a sulfur source to obtain a trisulfide compound (Step 3).

Examples of solvents used in Step 1 and Step 1' include methylene chloride, chloroform, and tetrahydrofuran, and methylene chloride is preferred. The amount of solvent may be 1 mL to 200 mL, preferably 3 mL to 35 mL, per 1 g of compound (1a) or compound (1b). Examples of condensing agents used in Step 1 and Step 1' include 1-(3-dimethylaminopropyl)-3-ethylcarbodiimide (EDC) and a salt thereof, N,N'-dicyclohexylcarbodiimide (DCC), diisopropylcarbodiimide (DIC), 4-dimethylaminopyridine (DMAP), and 1,1'-carbonyl diimidazole di(1H-imidazole-1-yl)methanone (CDI). When using these compounds as condensing agents, N-hydroxysuccinimide (NHS), 1-hydroxybenzotriazole (HOBt), etc. may be used as additives. The amount of condensing agent used in Step 1 and Step 1' may be 0.8 equivalents to 2.0 equivalents, preferably 1.0 equivalent to 1.5 equivalents, per 1 equivalent of compound (1a) or compound (1b). The reaction temperature in Step 1 and Step 1' may be -10°C to 40°C, preferably 15°C to 25°C. The reaction time in Step 1 and Step 1' may be 1 hour to 3 days, preferably 1 hour to 24 hours.

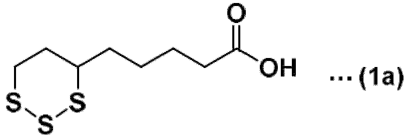

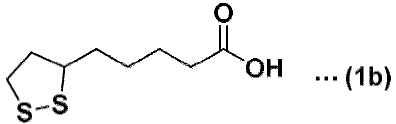

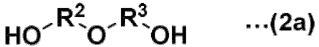

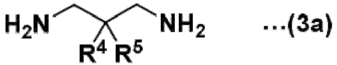

To link two molecules of compound (1a) or compound (1b) via a linker, the amount of the molecule for forming the linker used in Step 1 and Step 1' may be 0.45 to 1.00 equivalents relative to the amount of lipoic acid trisulfide. However, other equivalents may be used as long as the desired compound can be obtained.

The compound represented by Formula (1a) (lipoic acid trisulfide) can be produced by the methods described in Patent Literatures 2 and 3. The procedures and conditions for Steps 2 and 3 in production method (B) can be, for example, in accordance with the procedures and conditions for Steps 1 and 2 in Patent Literature 3.

R² and R³ in Formula (2a) are the same as R² and R³ in Formula (2), respectively. Examples of compounds represented by Formula (2a) include C₄₋₆ dialkyleneglycols. Examples of compounds represented by Formula (2a) include diethylene glycol, linear or branched dipropyleneglycol, and the like.

R⁴ and R⁵ in Formula (3a) are the same as R⁴ and R⁵ in Formula (3), respectively. Examples of compounds represented by Formula (3a) include 2,2-dimethyl-1,3-propanediamine, 2-oxetane-1,3-propanediamine, 2-methoxy-1,3-propanediamine, and the like.

Examples of compound (1) include compounds represented by the following Formulae (11) to (16) ("compounds (11) to (16)").

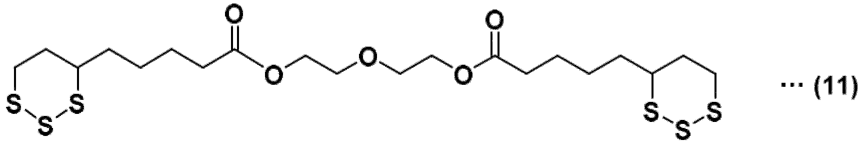

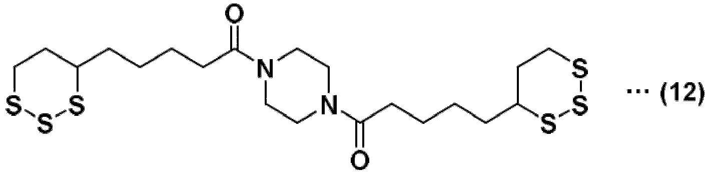

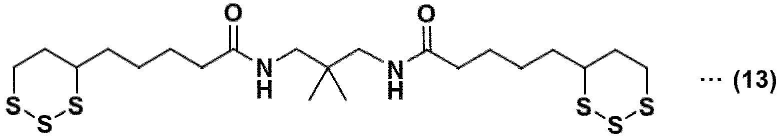

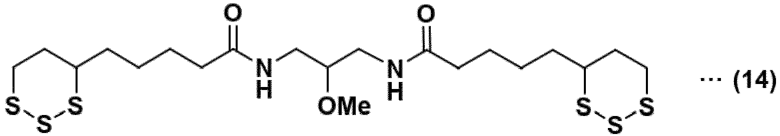

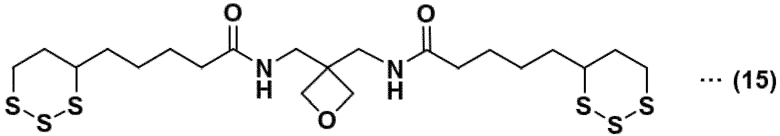

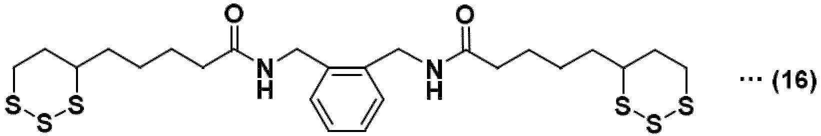

The molecular weights and ClogP values of compounds (11) to (16) are shown in Table 1.

**Table 1]**

| | Compound (11) | Compound (12) | Compound (13) | Compound (14) | Compound (15) | Compound (16) |
|---|---|---|---|---|---|---|
| M.W. | 546.8 | 526.9 | 542.9 | 544.9 | 556.9 | 576.9 |
| ClogP | 6.512 | 4.792 | 5.585 | 4.568 | 3.381 | 5.732 |

The molecular weight of compound (1) or a salt thereof may be 800 or less, 750 or less, or 700 or less, and is preferably 600 or less. The molecular weight of compound (1) or a salt thereof may be 400 or more, 450 or more, or 500 or more. When the molecular weight of compound (1) or a salt thereof is within these ranges, the *in vivo* absorption rate is considered to be further improved.

The ClogP of compound (1) or a salt thereof may be 1 or more, 2 or more, 3 or more, or 4 or more. The ClogP of compound (1) or a salt thereof may be 8 or less, 7 or less, or 6 or less. When the ClogP of compound (1) or a salt thereof is within these ranges, the balance of water solubility and lipophilicity of compound (1) or a salt thereof is considered to be more excellent. ClogP is a computer-calculated partition coefficient and can be determined according to the principles described in "CLOGP Reference Manual Daylight Version 4.9 (Release date: August 1, 2011, https://www.daylight.com/dayhtml/doc/clogp/)".

A salt of compound (1) may be a pharmacologically acceptable salt, and examples thereof include salts with alkali metals such as sodium and potassium; salts with alkaline earth metals such as calcium and magnesium; ammonium salts; salts with inorganic acids such as hydrochloric acid, hydrobromic acid, sulfuric acid, nitric acid, and phosphoric acid; and salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid. When compound (1) is obtained as a free form, it can be converted to a salt by a conventional method. When compound (1) is obtained as a salt, it can also be converted to a free form by a conventional method.

The compounds described in this specification may have four optical isomers: R,R-form, R,S-form, S,R-form, and S,S-form. The compounds described herein may be replaced with their optical isomers or mixtures containing the optical isomers at any ratio (for example, racemates). For example, compound (1) may be an optical isomer represented by Formula (1-1), an optical isomer represented by Formula (1-2), an optical isomer represented by Formula (1-3), or an optical isomer represented by Formula (1-4), or may be replaced with a mixture containing these optical isomers at any ratio (for example, racemate).

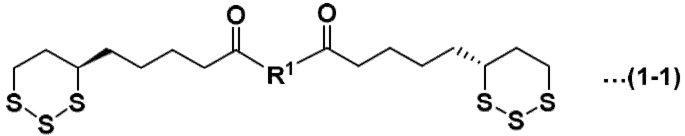

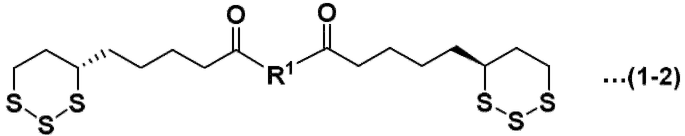

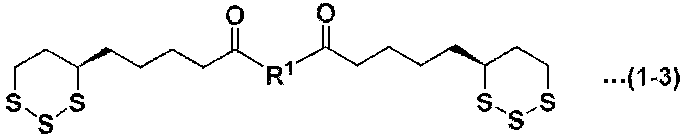

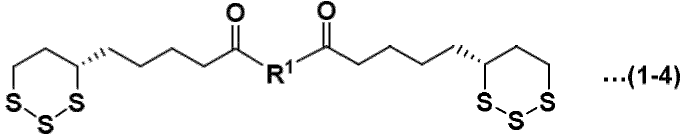

A CD clathrate according to another embodiment of the present invention is a compound in which at least one selected from the group consisting of compound (1) and a salt thereof is included in a cyclodextrin.

The cyclodextrin may be α-cyclodextrin, β-cyclodextrin, γ-cyclodextrin, or a derivative thereof. Here, "derivative" means that a hydrogen atom of at least one hydroxyl group of each cyclodextrin is substituted with an alkyl group optionally having a substituent or a saccharide. Examples of cyclodextrin derivatives include methyl-α-cyclodextrin, methyl-β-cyclodextrin, methyl-γ-cyclodextrin, dimethyl-α-cyclodextrin, dimethyl-β-cyclodextrin, dimethyl-γ-cyclodextrin, hydroxyethyl-α-cyclodextrin, hydroxyethyl-β-cyclodextrin, hydroxyethyl-γ-cyclodextrin, 2-hydroxypropyl-α-cyclodextrin, 2-hydroxypropyl-β-cyclodextrin, 2-hydroxypropyl-γ-cyclodextrin, glucosyl-α-cyclodextrin, glucosyl-β-cyclodextrin, glucosyl-γ-cyclodextrin, maltosyl-α-cyclodextrin, maltosyl-β-cyclodextrin, maltosyl-γ-cyclodextrin, sulfobutyl ether-α-cyclodextrin, sulfobutyl ether-β-cyclodextrin, and sulfobutyl ether-γ-cyclodextrin.

The CD clathrate can be produced by the method described in Patent Literature 3. More specifically, the CD clathrate can be produced by a step (Step a) of dissolving cyclodextrin in a solvent, a step (Step b) of adding a trisulfide compound or a salt thereof to the obtained solution and stirring, and a step (Step c) of filtering the stirred solution, washing with the same solvent as used in Step a, freezing the filtrate, and freeze-drying. The filtration and washing operation in Step c may be omitted.

Compound (1), a salt thereof, or a CD clathrate thereof can be formulated as a pharmaceutical composition by adding pharmacologically acceptable additives as necessary.

A pharmaceutical composition comprising compound (1), a salt thereof, or a CD clathrate thereof can be formulated as, for example, an injection, an oral agent, an eye drop, a nasal drop, an inhalant, an oral retention agent, a topical agent, a suppository, and the like. Examples of injections include subcutaneous injections, intramuscular injections, intravenous injections, and intraperitoneal injections. Examples of oral agents include tablets, granules, fine granules, powders, and capsules. Examples of eye drops include aqueous eye drops and oily eye drops. Examples of nasal drops include aqueous nasal drops and oily nasal drops. Examples of inhalants include aqueous inhalants and dry powder inhalants. Examples of oral retention agents include troches, sublingual tablets, and orally disintegrating tablets (OD tablets). Examples of topical agents include plasters, ointments, creams, lotions, and patches. Examples of suppositories include oleaginous base agent-type suppositories and water-soluble base material-type suppositories. Examples of additives include stabilizers such as saccharides (sucrose, trehalose, maltose, lactose, etc.), sugar alcohols (sorbitol, etc.), amino acids (L-arginine, etc.), water-soluble polymers (hydroxyethyl starch (HES), polyvinylpyrrolidone (PVP), etc.), nonionic surfactants (polysorbate, poloxamer, etc.), pH adjusters such as sodium phosphate buffer and histidine buffer, isotonic agents such as sodium chloride, and excipients such as mannitol, glycine, sodium chloride, and sucrose.

### [Examples]

Hereinafter, the present invention will be described in detail with reference to examples, but the present invention is not limited by these examples.

### Example 1

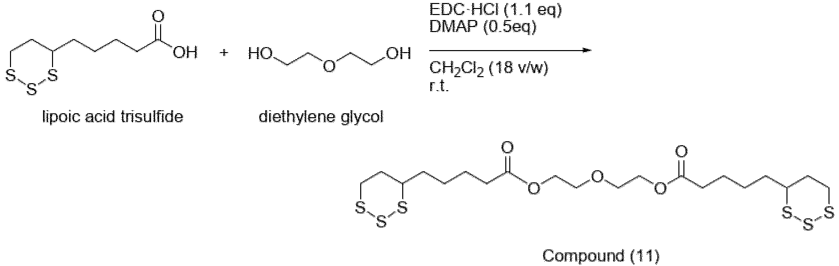

100 mg (0.42 mmol) of lipoic acid trisulfide and 1 mL (10 v/w) of methylene chloride were charged into a 5 mL round-bottom flask. After confirming that the contents of the flask had dissolved, 25.6 mg (0.21 mmol, 0.5 equivalents) of 4-dimethylaminopyridine (DMAP) and 88.5 mg (0.46 mmol, 1.1 equivalents) of 1-(3-dimethylaminopropyl)-3-ethylcarbodiimide hydrochloride (EDC HCl), and 0.4 mL (4 v/w) of methylene chloride were added. The mixture was then cooled to 0°C, and 44.5 mg (0.42 mmol, 1.0 equivalent) of diethylene glycol (DEG) and 0.4 mL (4 v/w) of methylene chloride were added. After replacing the air in the flask with nitrogen, the mixture was allowed to react overnight at room temperature. Thereafter, at room temperature, the organic layer was washed three times with 2 mL (20 v/w) of 1 mol/L hydrochloric acid aqueous solution, once with 2 mL (20 v/w) of 10% sodium hydrogen carbonate aqueous solution, and once with 2 mL (20 v/w) of brine. The organic layer was dried over sodium sulfate and filtered. The obtained filtrate was concentrated under reduced pressure, and the concentrated residue was purified by column chromatography (mobile phase: hexane/ethyl acetate mixture). The obtained fraction was concentrated under reduced pressure to give 16.7 mg (0.03 mmol, yield 15%, yellow oil) of compound (11).
¹H-NMR: (CDCl₃, 400 MHz) δ (ppm) = 4.23 (t, 4H, J = 4.8 Hz), 3.69 (t, 4H, J = 4.8 Hz), 3.51-3.13 (m, 6H), 2.35 (t, 4H, J = 8.0 Hz), 2.28-2.11 (m, 2H), 1.91 (tt, 2H, J = 13.2, 11.6 Hz), 1.72-1.40 (m, 12H)

### Example 2

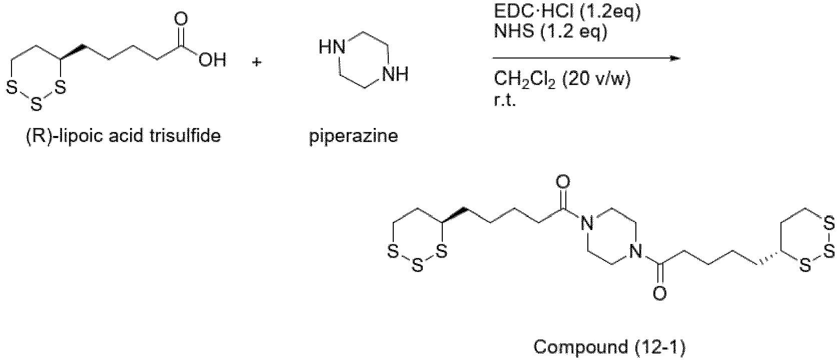

200 mg (0.84 mmol) of (R)-lipoic acid trisulfide and 4 mL (20 v/w) of methylene chloride were charged into a 25 mL round-bottom flask and cooled to 0°C. After confirming that the contents of the flask had dissolved, 193 mg (1.01 mmol, 1.2 equivalents) of 1-(3-dimethylaminopropyl)-3-ethylcarbodiimide hydrochloride (EDC HCl), 116 mg (1.01 mmol, 1.2 equivalents) of N-hydroxysuccinimide (NHS), and 32.5 mg (0.38 mmol, 0.45 equivalents) of piperazine were added to the flask. After replacing the air in the flask with nitrogen, the mixture was stirred overnight at room temperature. Thereafter, at room temperature, the organic layer was washed twice with 4 mL (20 v/w) of 10% sodium hydrogen carbonate aqueous solution and once with 4 mL (20 v/w) of brine. The obtained organic layer was concentrated under reduced pressure at an external temperature of 30°C, and the concentrated residue was purified by column chromatography (mobile phase: methylene chloride/methanol mixture). The fraction was concentrated under reduced pressure to give 58 mg (0.11 mmol, yield 27%, white solid) of compound (12-1).
1H-NMR: (CDCl3, 400 MHz) δ (ppm) = 3.64-3.60 (m, 4H), 3.47-3.43 (m, 4H), 3.34-3.16 (m, 6H), 2.34 (t, 4H, J = 7.2 Hz), 2.30-2.10 (m, 2H), 1.90 (tt, 2H, J = 12.8, 12.0 Hz), 1.67-1.44 (m, 12H)
ESI-TOF-MS: m/z 527.1051 ([M+H]⁺), calcd for [C₂₀H₃₅N₂O₂S₆] 527.1023

### Example 3

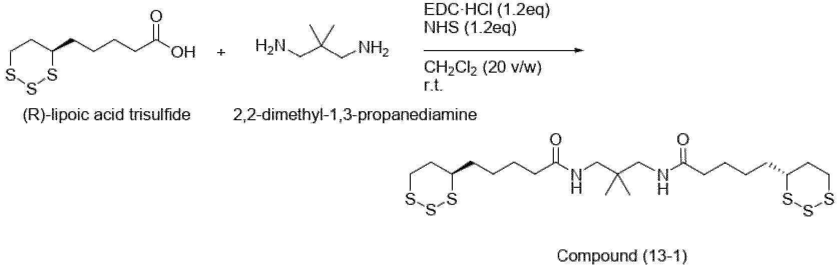

200 mg (0.84 mmol) of (R)-lipoic acid trisulfide and 4 mL (20 v/w) of methylene chloride were charged into a 25 mL round-bottom flask and cooled to 0°C. After confirming that the contents of the flask had dissolved, 193 mg (1.01 mmol, 1.2 equivalents) of 1-(3-dimethylaminopropyl)-3-ethylcarbodiimide hydrochloride (EDC HCl), 116 mg (1.01 mmol, 1.2 equivalents) of N-hydroxysuccinimide (NHS), and 45 µL (0.38 mmol, 0.45 equivalents) of 2,2-dimethyl-1,3-propanediamine were added to the flask. After replacing the air in the flask with nitrogen, the mixture was stirred overnight at room temperature. Thereafter, at room temperature, the organic layer was washed twice with 4 mL (20 v/w) of 10% sodium hydrogen carbonate aqueous solution. The obtained organic layer was concentrated under reduced pressure at an external temperature of 30°C, and the concentrated residue was purified by column chromatography (mobile phase: methylene chloride/methanol mixture). The fraction was concentrated under reduced pressure to give 112 mg (0.21 mmol, yield 51%, white solid) of compound (13-1).
¹H-NMR: (CDCl₃, 400 MHz) δ (ppm) = 6.54 (t, 2H, J = 6.4 Hz), 3.35-3.04 (m, 6H), 2.98 (d, 4H, J = 6.4 Hz), 2.25-2.17 (m, 6H), 1.89 (tt, 2H, J = 12.4, 12.0 Hz), 1.66-1.43 (m, 12H), 0.86 (s, 6H)
ESI-TOF-MS: m/z 543.1389 ([M+H]⁺), calcd for [C₂₁H₃₉N₂O₂S₆] 543.1336

### Test Example 1

The thermal stability of (R)-α-lipoic acid, (R)-lipoic acid trisulfide, and compound (12-1) was evaluated as follows. Each compound was placed in a round-bottom flask and heated at 100°C for 5 hours. Every hour, a sample was taken from the flask, dissolved in a methylene chloride/methanol mixture (1:1), and subjected to HPLC. The residual rate over time was calculated from the area values of each component.

### Test Results

The results are shown in Fig. 1. In Fig. 1, the residual rate on the vertical axis was calculated from the degree of decomposition over time for each sample, with the amount before heating set at 100%. (R)-α-lipoic acid had a residual rate below 60% at 1 hour, and then gradually decomposed to about 50% after 5 hours. (R)-lipoic acid trisulfide showed no decomposition at 2 hours, but gradually decomposed after 3 hours, with a residual rate of about 70% after 5 hours. Compound (12-1) showed almost no decomposition even after 5 hours, maintaining a residual rate of 95% or more. In addition, (R)-α-lipoic acid and (R)-lipoic acid trisulfide melted immediately after the start of heating, and insoluble by-products were formed. However, compound (12-1) did not melt and remained solid. This is presumed to be due to the high melting point of compound (12-1) and suppression of intermolecular polymerization. From the above, it was confirmed that the compound or salt represented by Formula (1) has higher thermal stability than (R)-α-lipoic acid and (R)-lipoic acid trisulfide.

## Claims

1. A compound represented by the Formula (1) or a salt thereof: 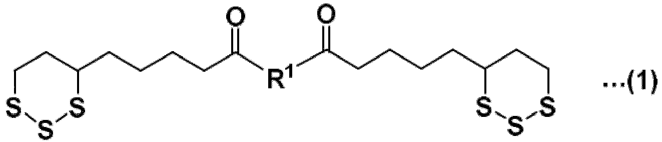
wherein R¹ is a group represented by the Formula (2): 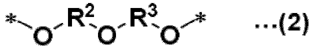
wherein R² and R³ are each independently a C₁₋₄ alkylene group, and * is a bond;
a group represented by the Formula (3): 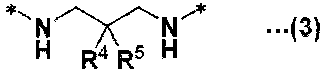
wherein R⁴ and R⁵ are each independently a hydrogen atom, a C₁₋₄ alkyl group optionally having a substituent, R⁴ and R⁵ may form a ring together with the atom bonded thereto, and * is a bond;
a group represented by the Formula (4): 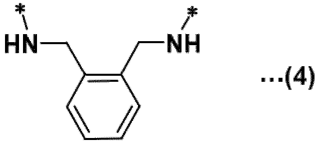
wherein * is a bond; or
a group represented by the Formula (5): 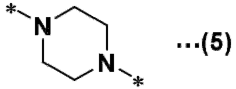
wherein * is a bond.

2. A cyclodextrin clathrate wherein the compound or salt thereof according to claim 1 is included in a cyclodextrin.

3. The clathrate according to claim 2, wherein the salt of the compound represented by Formula (1) comprises at least one selected from the group consisting of a salt with an alkali metal, a salt with an alkaline earth metal, an annnonium salt, a salt with an inorganic acid, and a salt with an organic acid.

4. The clathrate according to claim 2 or 3, wherein the cyclodextrin is at least one selected from the group consisting of α-cyclodextrin, β-cyclodextrin, γ-cyclodextrin, and a derivative thereof.
